# EUROPEAN PATENT SPECIFICATION

(11) **EP 2 792 380 B1**
(45) Date of publication and mention of the grant of the patent: **13.12.2017**
(21) Application number: 14001350.9
(22) Date of filing: 14.04.2014
(51) Int. Cl.: A61M 5/31, A61L 31/08, B05D 1/00, B05D 7/00, B65D 83/00, C08J 7/04, C23C 16/00, A61M 39/08, A61B 5/15, A61B 5/153, B05D 7/02

(54) **Use of acrylic acid for hydrophilically coating a medicinal-technical device**
Verwendung von Acrylsäure zum hydrophilen Beschichten einer medizintechnischen Vorrichtung
Utilisation d'acide acrylique pour revêtement hydrophile d'un dispositif technique médicinal

(30) Priority: 15.04.2013 EP 13001970; 03.02.2014 WO PCT/EP2014/000277
(43) Date of publication of application: 22.10.2014
(73) Proprietor: ABAG Aktienmarkt Beteiligungs AG, 50668 Köln (DE)
(72) Inventor: Görne, Martin, 22337 Hamburg (DE)
(74) Representative: Diehl & Partner GbR

(56) References cited:
- EP-A1- 2 705 859
- WO-A1-97/22631
- WO-A1-2014/064299

## Description

The present invention relates to hydrophilically coated medicinal-technical devices in general, and in particular to an inwardly hydrophilically coated medicinal-technical device for temporally accommodating therein a bodily liquid, in particular blood, or a cell suspension. In particular, the device may be a bottle, a bag, a tube, or a pipe, but may also be an actuatable part of an apparatus for applying an external action, such as pressure, heat or radiation, on the bodily liquid or blood or cell suspension, respectively. The coating is made using acrylic acid or its anhydride in a combined PECVD/CVD process.

Devices for temporally accommodating blood are known, some of which are for flowing through and some of which are for temporal storage. As an example, the blood tube disclosed in the German laid-open patent application DE 10 2011 108 787 A1 may be mentioned, the content of which is herein disclosed by reference insofar relating to the arrangement of a blood guiding system.

A general phenomenon of such devices is the increase of the tendency of the blood, or certain protein components in the bodily liquid, to coagulate due to the contact with the device. This effect in most applications is undesirable, and is counteracted, if necessary, by the administration of agents reducing the coagulation tendency. On the other hand, such agents have, as such, undesirable side effects; be it that blood preservations are used during a surgical operation, and the surgeon desires speedy stoppage of bleeding; or be it that a bodily liquid is desired to be returned or further processed as purely and uncontaminated as possible. Similar issues are encountered with cell suspensions intended for being administered to a human being or a vertebrate animal (or mammal). Document WO 97/22631 A1 discloses generating a three-dimensional film network comprising a plurality of radio frequency discharge plasma film layers including at least a first layer, comprising a plurality of a first functional group, such as an amine, and a second layer, comprising a plurality of a second functional group different from the first functional group, such as a carboxylic acid.

It is known from the patent application US 2012/0123345 A1 to coat inner surfaces of vessels by inserting an electrode in the hollow space within the vessel, and igniting a plasma in the presence of a reactive gas. The (hollow) electrode simultaneously serves as a gas supply, while the (concave) counter electrode is arranged surrounding the vessel. With respect to the arrangement of the apparatus components, this patent application is likewise incorporated herein by reference. The express purpose of this known method is to provide water-repellent surfaces. The inwardly coated vessels obtained in this manner are, however, in practice not yet satisfactory for temporary accommodation of bodily liquids, blood, or cell suspensions, respectively.

The present invention aims at diminishing, reducing or avoiding the disadvantages of the known devices and methods. To this end, the invention provides a use of acrylic acid or acrylic acid anhydride for hydrophilizing surfaces of a body, according to claim 1.

A hollow body treated by the use according to the invention has, at its inner surface, a hydrophilizing coating. Thereby, the tendency of the bodily liquid, blood, or cell suspension flown therethrough or accommodated therein is increased less than hitherto possible. In particular, a water contact angle, which designates the degree of hydrophily of a surface, may be set to less than 2° or even less than 0.5°. In embodiments, the surface material of the hollow body is a synthetic resin, and the hydrophilizing layer is made up of acrylic acid units. The hollow body may have exactly one opening, and may be a bottle or a bag; but it may also have two openings, through which, in use, the blood, bodily liquid, or cell suspension, respectively, is flown, and may be a pipe or a tube; or it may be an actuatable part of an apparatus applying, in operation, some external action, such as pressure, heat, or radiation, on the bodily liquid, blood, or cell suspension. If the body has any other shape, such as convex, it may still be a part or component e.g. intended for use inside a hollow body for temporarily storing bodily liquids or cell suspensions. Naturally, even a convex body (cup, torus, hourglass shaped) may have indented, or concave surface regions; such a body may be termed "generally convex". Such generally convex bodies may be rendered hydrophilic by the invention even if not intended for use inside a hollow body for temporarily storing bodily liquids or all suspensions.

The process of coating includes a pre-treating step and a follow-up-treating step. Herein, in the pre-treating step, both inert gas as also a reactive gas is present; and in the terminal follow-up-treating step, substantially only the reactive gas is present, and there is no plasma action. The pre-treating may take less than 1 minute; the surface material of the hollow body may be made of a synthetic resin; and the coating material may be acrylic acid or its anhydride. In embodiments, the pre-treating may also take between 1 minute and less than 10 minutes.

Without wishing to be bound by theory, it is assumed that the particularly short duration of the pre-treating step according to present invention allows for activated sites on the surface not to be immediately de-activated by the plasma, so that the two-step PECVD/CVD-coating particularly efficiently results in a hydrophilic surface.

Further advantages and features of the present invention will become apparent from the following detailed description of embodiments in conjunction with the drawings. The invention is not limited to the described embodiments, but by the scope of the appended claims. In particular, individual feature may be realized in embodiments according to the invention in a different number or und combination as in the examples described below. In the subsequent explanation of embodiments of the invention, reference is made to the accompanying drawings, which show:
- Figure 1: a schematic cross-section through an inwardly coated hollow body, and
- Figure 2: a schematic flow diagram of a process of inwardly coating a hollow body.

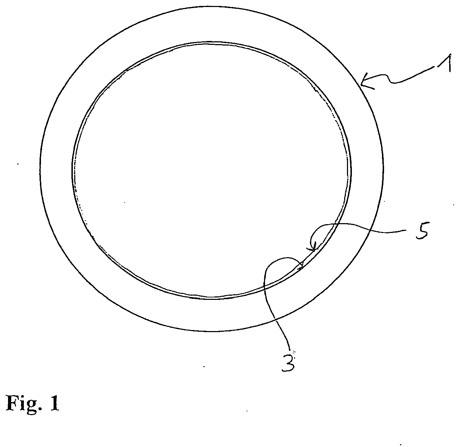

Figure 1 depicts a generally oval, in the embodiment shown: substantially circular cross section of the hollow body 1. Although the wall is shown as being unitary and seamless, it may in actual fact be multi-layered and/or may have one or more seams. The inner surface layer 3 is formed of a synthetic resin, which term shall designate cross-linked or non-cross-linked polymeric materials, the latter in particular in conjunction with two-(or more)layer walls, in which the mechanical stability is provided mainly by the outer layer or layers. The water contact angle of the inner surface layer is 10° or more, in particular 30° or more, and the surface layer will be termed *"little hydrophilic"* in this context. Through the combined PECVD/CVD-coating process with ultra-short pre-coating, described in detail below, a very thin adhesion layer is formed on the inner surface and on top of that, a thicker function layer 5, both made of carboxy group-containing units, e. g. acrylic acid units. These layers are stably bound to the inner surface layer and are surprisingly extremely hydrophilic, notably more hydrophilic than those obtained in an otherwise similar manner, but with a longer (> 1 minute, e.g. 10 minutes or more) pre-coating phase. It is thought that a longer pre-coating lets active sites, once generated, react further or with one another, in a manner detrimental to the hydrophilicity so obtainable. At the same time, a certain action time of the plasma is required for achieving a stable coating, such as at least 2 seconds.

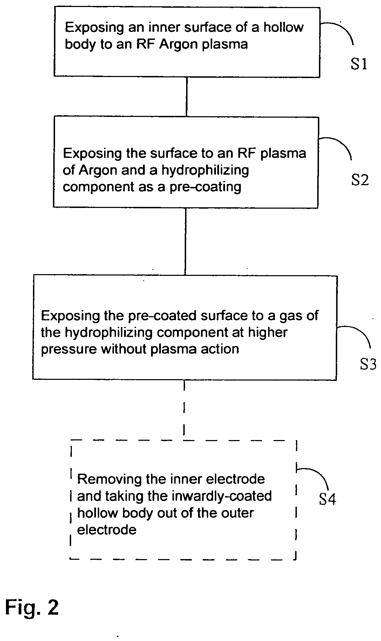

The flow diagram scheme of Figure 2 illustrates the important steps of a process for hydrophilizing inner surfaces by coating with poly(acrylic acid): After positioning a hollow electrode in the vessel or one-sidedly closed pipe or tube, same is evacuated by means of pumps, preferably to a pressure of maximally 10⁻⁴ mbar (10 mPa). After reaching the desired vacuum pressure, the interior of the vessel is purged with an inert gas, preferably Argon, while continuously pumping, wherein the inert gas supply is adjusted to the pumping speed so that a constant gas pressure is maintained in the interior of the vessel. The inert gas is supplied from an inert gas reservoir. In preferred embodiments, the Argon pressure so adjusted is about 25-150 mTorr (3.3-20 Pa). After reaching a stable inert gas pressure in the interior of the vessel, the plasma generator, for example a high frequency generator, is switched on for generating an inert gas plasma. The plasma cleans the inner surfaces by removing substances adsorbed thereto, and furthermore results in an activation of the inner surfaces by the formation of ions and free radicals, which enhance the subsequent graft polymerizing step. In some other embodiments, this pre-treatment step can be dispensed with.

The cleaning and activating effect in this first step S1 may be influenced *via* the frequency of the gas plasmas, the power fed into the plasma, the exposition time to the plasma and the kind of the inert gas used for the plasma. As the inert gas, in the process proposed here Argon is preferred, because it allows for an activation of the inner surfaces without generating new, undesired compounds. Naturally, instead other inert gases such as N₂ may be used. In an exemplary embodiment of the process, the exposition time to the Argon plasma is 20 to 120 seconds. After the exposition, the process is continued with the first coating step S2. The plasma generator may be switched off temporarily if desired.

Deviating from the above, the plasma may be generated on the basis of a mixture of the inert gas and a reactive component used in a subsequent pre-coating step, instead of on the basis of pure Argon. The partial pressure of the reactive component in the gas mixture should in this case be less than one tenth of the partial pressure of the inert gas.

On transitioning from step S1 to step S2 of the process, the inert gas supply into the interior of the vessel is preferably maintained and desirably adjusted so that it assumes a value suitable for carrying out step S2. For generating the gas mixture, a first coating material gas formed of carboxy group-containing monomers in the gas phase is admixed to the inert gas. The carboxy group-containing monomers may preferably be acrylic acid or a precursor of acrylic acid, such as e.g. acrylic acid anhydride. The partial pressure p_{eSG} of the first coating material gas in embodiments is at least one fourth of and maximally twice the partial pressure p_{IG} of the inert gas. It is particularly preferred that the partial pressure ratio p_{eSG}:p_{IG} is selected from a range of 1:1 to 1:0.5. For example, the partial pressure of Argon in embodiments of the process is 30 mTorr (ca. 400 mPa) at a total pressure of the gas mixture of 45 mTorr (ca. 600 mPa), resulting in a value of 2:1 for the ratio of the Argon partial pressure p_{Ar} to the partial pressure of the first coating material gas (reactive component partial pressure) p_{eSG}. While adjusting the desired reactive gas mixture the plasma generator may temporarily be switched off.

As the reactive component for forming the first coating material gas, preferably (meth)acrylic acid anhydride is used, which is evaporated in a separate container and is then guided to the interior of the vessel. The partial pressure of the coating material gas is adjusted via its supply, controlled by means of valves. Naturally, instead of (meth) acrylic acid anhydride (meth)acrylic acid may be used. (Meth)acrylic acid or (meth) acrylic acid anhydride, respectively, are provided in reservoir containers in liquid form, for example in an amount of 150 mL. In order to avoid or inhibit polymerisation of the acrylic acid or its precursor materials, respectively, same may be doted with Cu(I)-chloride. Furthermore, the reactive component containers are de-aerated after filling until no bubbles form in the reactive component liquid any longer. The vapour pressure of the reactive components usually is sufficient at normal room temperatures of 22 to 25°C for generating the first coating material gas.

After the desired gas mixture and gas mixture pressure have been formed, which typically takes 1 to 2 minutes, the actual coating process is initiated by starting the plasma generator, whereby excited acrylic acid monomers generated in the plasma attach to the activated inner surface and, in the subsequent process, form a poly(acrylic acid) layer. This plasma-enhanced pre-coating phase (PECVD) is maintained only for a short time in order to form a very thin adhesion layer. The pre-coating phase may take less than 60, or between 2 and 20 seconds, resulting in a particularly low contact angle for water such as below 2° or even below 0.5°. The gas supplies are preferably not varied during the plasma coating. In a first variant of the process, the pre-coating process is terminated by switching off the plasma generator.

Subsequent to the described first variant of the pre-coating step S2, a first variant of the follow-up-coating step S3 follows, in which after switching off the plasma generator initially the inert gas supply is interrupted and the pre-coated inner surface is exposed to, if possible, the full vapour pressure of the reactive component formed of water-free acrylic acid. This vapour pressure should not be below ca. 5 Torr (670 Pa). Slightly cooling or warming the reactive component in the reservoir container may be suitable for adjusting the pressure. Supplying the reactive component into the interior of the vessel at full vapour pressure provides reactive gas in large amounts, which gas reacts with the reactive centres present at the pre-coated surface and forms a relatively thick poly(acrylic acid) layer (PAA-layer) which may be crystalline.

In a second variant of the process, the plasma generator is not switched off at the end of the pre-coating step S2 and is therefore still in operation at the transition to the second variant of the follow-up-coating step S3. In this variant, the Argon supply is stopped almost or entirely, and the supply of the reactive gas, e. g. of the acrylic acid, is reduced to such an extent that, while maintaining high-frequency generation and continuously evacuating the interior of the vessel, a pressure equilibrium in the range of less than 0.3 mTorr (ca. 40 mPa) is adjusted. In an exemplary embodiment, the pressure is set to a pressure of less than 0.1 mTorr (ca. 13 mPa). Thereby, the plasma extinguishes due to the reduction in pressure. Otherwise, the second variant is similar to the first variant. In both variants, the follow-up-coating phase is maintained for 1 to 45, or for 5 to 15 minutes. After termination of the process in step S4, the inwardly coated vessels can be taken out of the apparatus along with the hollow electrode, and the inward coating may be inspected for quality evaluation, if desired.

The process described above allows for a long-term-stable hydrophilization of polymeric inner surface of hollow bodies or vessels, respectively, having a low tendency of coagulation of the bodily liquids or cell suspensions flown therethrough, or temporarily stored therein, or acted upon externally while in the hollow body.

In another embodiment particularly suited for hydrophilically coating generally convex bodies, or even for coating hollow bodies on their outer surface, the bodies are arranged, e.g. side-by-side, on a wire grid, so that their surface is readily accessible from the gas phase for coating. The wire grid may at the same time serve as one of the electrodes used for generating the plasma. Other wire-based electrode shapes are also possible. In order to ascertain a fairly homogenous electric field, a flat wire grid is often suitable. As the counter electrode, another wire gird (flat or having another shape) may be used. In some embodiments, the bodies are arranged between two such wire grids, e.g. on the grounded one; while in other embodiments, the bodies are arranged on the upper one of two such wire grids. Several such pairs of electrodes may be arranged as a stack.

The skilled person will realize that numerous modifications and augmentations to the above described embodiments are possible. For example, while the above description advocates an initial pre-treatment step, such step may be dispensed with if the surfaces are already sufficiently clean. In such a case, the initial step is the pre-coating step, and there is no preceding step in which the amount (or partial pressure) of reactive gas is less than one tenth of that of the inert gas, or in which the reactive gas is even absent. Further, while the above process has two distinct steps, similar results may be obtained by a gradual transition. to the plasma-less phase, resulting in a more gradual structure change of the deposit layer. The skilled person will appreciate that while in the above description of the apparatus used for inwardly coating a hollow body, the counter electrode is located outside the hollow body, it will also be possible to accommodate both electrodes inside the body, if the counter electrode is formed as a metallic rod or tube. The tip of the electrode serving as the gas supply may be of the showerhead-type. Alternatively, in cases where at least the surface of the hollow body is electrically conductive, such as metallic, it will be possible to arrange the hollow body on e.g. a ring-like structure which is grounded. In this case, only one metallic tube having a gas outlet is required to enter into the hollow body, while the outer surface of the hollow body may serve as the counter electrode. Naturally, the two electrodes are insulated from one another; where two electrodes are inserted into the hollow body, one of them may be supported by a ring-like structure.

## Claims

1. Use of acrylic acid or acrylic acid anhydride for hydrophilizing surfaces of a body, the use comprising:
- pre-coating by PECVD the surfaces using a high frequency plasma, wherein the pre-coating by PECVD takes less than 10 minutes; and then
- follow-up coating the pre-coated surfaces of the body,
**characterized in that** the pre-coating uses a high frequency plasma generated from a gas mixture composed of an inert gas and a first gas formed of biocompatible, polymerizable, carboxy group-containing monomers, and **in that** the follow-up coating is made by CVD without plasma action using a second gas substantially consisting of the acrylic acid or acrylic acid anhydride monomers.

2. The use of claim 1, wherein the pre-coating takes less than 1 minute.

3. The use of claim 2, wherein the pre-coating PECVD step takes 2-20 seconds.

4. The use of one of the preceding claims, consisting of the pre-coating PECVD step and the follow-up coating CVD step without plasma action.

5. The use of one of the preceding claims, wherein the body is generally convex, and the surfaces so coated are outer surfaces of the body.

6. The use of claim 5, wherein the pre-coating comprises supporting the generally convex body on a wire-grid serving as an electrode for generating the plasma.

7. The use of claim 6, wherein the wire-grid is generally flat.

8. The use of one of claims 5 to 7, wherein the use comprises using the generally convex body inside a hollow body for temporarily accommodating therein bodily liquids.

9. The use of one of claims 1 to 4, wherein the body is hollow, and the surfaces so coated are inner surfaces of the body.

10. The use of claim 9, wherein the pre-coating comprises inserting, into the hollow body, an inner electrode for generating the plasma.

11. The use of one of claims 8 to 10, further comprising temporarily accommodating in the hollow body a bodily liquid, in particular blood, or a cell suspension.

12. The use of claim 9, wherein the temporarily accommodating includes filling the bodily liquid or cell suspension into the hollow body, storing it, and later discharging it from the hollow body, such as in opposite direction to the filling.

13. The use of claim 11, wherein the temporarily accommodating includes steadily flowing the bodily liquid or cell suspension through the hollow body.

14. The use of one of claims 11 to 13, comprising applying an external action such as pressure, heat, or radiation on the bodily liquid or the cell suspension.

15. The use of one of the preceding claims, wherein the follow-up coating CVD step takes 1-45 minutes, in particular 5-15 minutes.

## Patentansprüche

1. Verwendung von Acrylsäure oder Acrylsäureanhydrid zum Hydrophilieren von Oberflächen eines Körpers, die Verwendung beinhaltend:
- Vorbeschichten der Oberflächen durch PECVD mittels eines Hochfrequenzplasmas, wobei das Vorbeschichten durch PECVD weniger als 10 Minuten dauert; und dann
- Folgebeschichten der vorbeschichteten Oberflächen des Körpers,
**dadurch gekennzeichnet, dass** das Vorbeschichten ein Hochfrequenzplasma verwendet, das aus einer Gasmischung erzeugt wird, die sich aus einem Inertgas und einem ersten Gas zusammensetzt, welches aus biokompatiblen, polymerisierbaren, Carboxylgruppen enthaltenden Monomeren gebildet ist, und dadurch, dass das Folgebeschichten durch CVD ohne Plasmaeinwirkung geschieht, unter Verwendung eines zweiten Gases, das im Wesentlichen aus den Acrylsäure oder Acrylsäureanhydrid-Monomeren besteht.

2. Verwendung nach Anspruch 1, wobei das Vorbeschichten weniger als 1 Minute dauert.

3. Verwendung nach Anspruch 2, wobei der vorbeschichtende PECVD-Schritt 2-20 Sekunden dauert.

4. Verwendung nach einem der vorstehenden Ansprüche, bestehend aus dem vorbeschichtenden PECVD-Schritt und dem folgebeschichtenden CVD-Schritt ohne Plasma-Einwirkung.

5. Verwendung nach einem der vorstehenden Ansprüche, wobei der Körper allgemein konvex ist, und die so beschichteten Oberflächen äußere Oberflächen des Körpers sind.

6. Verwendung nach Anspruch 5, wobei das Vorbeschichten das Haltern des allgemein konvexen Körpers auf einem Drahtgitter beinhaltet, welches als Elektrode zur Erzeugung des Plasmas dient.

7. Verwendung nach Anspruch 6, wobei das Drahtgitter allgemein flach ist.

8. Verwendung nach einem der Ansprüche 5 bis 7, wobei die Verwendung die Verwendung des allgemein konvexen Körpers innerhalb eines Hohlkörpers zur temporären Aufnahme einer Körperflüssigkeit beinhaltet.

9. Verwendung nach einem der Ansprüche 1 bis 4, wobei der Körper hohl ist, und die so beschichteten Oberflächen innere Oberflächen des Körpers sind.

10. Verwendung nach Anspruch 9, wobei das Vorbeschichten das Einführen einer inneren Elektrode zum Erzeugen des Plasmas in den Hohlkörper beinhaltet.

11. Verwendung nach einem der Ansprüche 8 bis 10, ferner beinhaltend temporäres Beherbergen einer Körperflüssigkeit, insbesondere Blut, oder einer Zellsuspension, in dem Hohlkörper.

12. Verwendung nach Anspruch 9, wobei das temporäre Beherbergen das Einfüllen der Körperflüssigkeit oder der Zellsuspension in den Hohlkörper, ihr Aufbewahren darin, und ihr späteres Entleeren aus dem Hohlkörper beinhaltet, wie etwa in Gegenrichtung zum Einfüllen.

13. Verwendung nach Anspruch 11, wobei das temporäre Beherbergen das ständige Strömen der Körperflüssigkeit oder der Zellsuspension durch den Hohlkörper beinhaltet.

14. Verwendung nach einem der Ansprüche 11 bis 13, beinhaltend das Ausüben einer äußeren Einwirkung wie Druck, Temperatur oder Strahlung auf die Körperflüssigkeit oder Zellsuspension.

15. Verwendung nach einem der vorstehenden Ansprüche, wobei der folgebeschichtende CVD-Schritt 1-45 Minuten, insbesondere 5-15 Minuten dauert.

## Revendications

1. Utilisation d'acide acrylique ou d'anhydride d'acide acrylique pour hydrophiliser des surfaces d'un corps, l'utilisation comprenant :
- le pré-revêtement par PECVD des surfaces en utilisant un plasma haute fréquence, dans laquelle le pré-revêtement par PECVD prend moins de 10 minutes ; puis
- le revêtement de suivi des surfaces pré-revêtues du corps,
**caractérisée en ce que** le pré-revêtement utilise un plasma haute fréquence généré à partir d'un mélange de gaz composé d'un gaz inerte et d'un premier gaz formé de monomères biocompatibles, polymérisables, contenant un groupe carboxy, et **en ce que** le revêtement de suivi est réalisé par CVD sans action de plasma en utilisant un second gaz consistant sensiblement en des monomères d'anhydride d'acide acrylique ou d'acide acrylique.

2. Utilisation selon la revendication 1, dans laquelle le pré-revêtement prend moins de 1 minute.

3. Utilisation selon la revendication 2, dans laquelle l'étape de pré-revêtement par PECVD prend de 2 à 20 secondes.

4. Utilisation selon l'une quelconque des revendications précédentes, consistant en l'étape de pré-revêtement par PECVD et l'étape de revêtement de suivi par CVD sans action de plasma.

5. Utilisation selon l'une quelconque des revendications précédentes, dans laquelle le corps est généralement convexe et les surfaces ainsi revêtues sont les surfaces externes du corps.

6. Utilisation selon la revendication 5, dans laquelle le pré-revêtement comprend le support du corps généralement convexe sur une grille de fils servant d'électrode pour générer le plasma.

7. Utilisation selon la revendication 6, dans laquelle la grille de fils est généralement plate.

8. Utilisation selon les revendications 5 à 7, dans laquelle l'utilisation comprend l'utilisation du corps généralement convexe à l'intérieur d'un corps creux pour accueillir temporairement dans celui-ci des liquides corporels.

9. Utilisation selon l'une quelconque des revendications 1 à 4, dans laquelle le corps est creux et les surfaces ainsi revêtues sont les surfaces internes du corps.

10. Utilisation selon la revendication 9, dans laquelle le pré-revêtement comprend l'insertion, dans le corps creux, d'une électrode interne pour générer le plasma.

11. Utilisation selon l'une quelconque des revendications 8 à 10, comprenant en outre l'accueil temporaire dans le corps creux d'un liquide corporel, en particulier du sang, ou une suspension de cellules.

12. Utilisation selon la revendication 9, dans laquelle l'accueil temporaire comporte le remplissage du liquide corporel ou de la suspension de cellules dans le corps creux, leur stockage et leur vidage ultérieur du corps creux, tel que dans une direction opposée au remplissage.

13. Utilisation selon la revendication 11, dans laquelle l'accueil temporaire comporte l'écoulement régulier du liquide corporel ou de la suspension de cellules à travers le corps creux.

14. Utilisation selon l'une quelconque des revendications 11 à 13, comprenant l'application d'une action externe telle qu'une pression, une chaleur ou un rayonnement sur le liquide corporel ou la suspension de cellules.

15. Utilisation selon l'une quelconque des revendications précédentes, dans laquelle l'étape de revêtement de suivi par CVD prend de 1 à 45 minutes, en particulier de 5 à 15 minutes.
